# EUROPEAN PATENT SPECIFICATION

(11) **EP 2 598 097 B1**
(45) Date of publication and mention of the grant of the patent: **22.08.2018**
(21) Application number: 11740773.4
(22) Date of filing: 25.07.2011
(51) Int. Cl.: A61F 13/56, A61F 13/62

(54) **ABSORBENT ARTICLE HAVING FASTENING MEMBERS WITH INDICIUM**
ABSORBIERENDER GEGENSTAND ENTHALTEND VERSCHLUSSELEMENTE MIT POSITIONIERMARKIERUNGEN
ARTICLE D'HYGIÈNE ABSORBANT COMPRENANT DES ÉLÉMENTS DE FERMETURE POURVUS DE REPÈRES DE FIXATION

(30) Priority: 27.07.2010 CA 2708725
(43) Date of publication of application: 05.06.2013
(73) Proprietor: The Procter & Gamble Company, Cincinnati, OH 45202 (US)
(72) Inventor: DOBRIN, George, Christopher, Mason Ohio 45040 (US)
(74) Representative: Kremer, Véronique Marie Joséphine
(86) International application number: PCT/US2011/045133
(87) International publication number: WO 2012/015710

(56) References cited:
- WO-A1-01/21126

## Description

### FIELD OF THE INVENTION

One aspect of the invention generally relates to fastening members including an indicium, and which are suitable for use in absorbent article. In one aspect, the invention relates to fastening members having an indicium, which resembles a macro-mechanical fastener and is visible on a garment facing surface of the fastening member. One particular aspect of the invention further relates to fastening members having an indicium, which resembles a macro-mechanical fastener that is visible on a garment facing surface of the fastening member and also includes an actual mechanical fastener disposed on a body facing surface of the fastening member.

### BACKGROUND OF THE INVENTION

The prior art is replete with fastening members for use in disposable absorbent articles that are positioned about the lower torso of a wearer. Disposable absorbent articles include diapers, training pants and the like. Typical fastening members are in the form of a tape disposed in one of the waist regions of the disposable absorbent article. The tape includes a fastener on one of its sides, typically the body facing side, for engaging a complementary or receiving member disposed on the other waist region of the disposable absorbent article. For example, the fastener may an adhesive, which provides suitable attachment of the absorbent article about the waist of a wearer when the adhesive is in contact with a film. Alternatively, the fastener may be a micro mechanical fastener such as a plurality of "hooks," which provide suitable attachment of the absorbent article when the hooks engage at least some of the fibers of a fabric such as a knitted or nonwoven material. The tape itself can be made of various materials such as one or more layers of a film, a fibrous nonwoven material, or laminates of such materials. Some of the tapes are substantially white, while others are tinted or include color for improved aesthetic. However, such tapes are typically opaque and do not allow the care giver to see the fastener through the tape. This may result in the improper positioning of the fastener by the care giver, in particular when the care giver is a new parent, on the complementary fastening surface or receiving member. The improper positioning of the fastener may itself cause the absorbent article to get detached from the wearer lower torso. It is therefore one object of the invention to provide a fastening member with an indicium, which is visible and can help a care giver identify the position of a fastener for its proper engagement to the complementary fastening surface or receiving member. It is a further object of the invention to provide an indicium that is aesthetically pleasing and improves the garment like appearance of the disposable absorbent article.

WO 01/211267A1 discloses an absorbent article, such as a disposable diaper, including side panels and fastener elements for assembling the diaper about the body of the wearer. The outer surface of the diaper and the side panels or fastener tabs include indicia that complement each other and are provided to facilitate the proper and symmetrical application of the diaper to the wearer for maximum comfort and for optimal functioning of the diaper.

### SUMMARY OF THE INVENTION

One aspect of the invention is directed to an absorbent article as defined in claim 1, with preferred aspects being defined in dependent claims 2-6.

### BRIEF DESCRIPTION OF THE DRAWINGS

While the specification concludes with claims particularly pointing out and distinctly claiming the subject matter that is regarded as the present invention, it is believed that the invention will be more fully understood from the following description taken in conjunction with the accompanying drawings. None of the drawings are necessarily to scale.
FIG. 1 is a schematic, garment facing surface view of a fastening member in accordance with one embodiment of the invention;
FIG. 2 is a schematic, body facing surface view of a fastening member in accordance with one embodiment of the invention of FIG. 1;
FIGS. 3A through 3E are schematic, garment facing surface view of a fastening member in accordance with other embodiments of the invention;
FIGS. 4A through 4B are schematic, garment facing surface view of fastening members having an arc-like edge;
FIGS. 5A through 5E arc schematic, cross-scctional view of fastening members in accordance with embodiments of the invention;
FIG. 6 is garment facing surface view of an absorbent article including a pair of fastening members in accordance with one embodiment of the invention; and
FIGS. 7A through 7C are schematic illustrations of a process for cutting a fastening member from a continuous strip of material(s) in accordance with embodiments of the invention.

### DETAILED DESCRIPTION OF THE INVENTION

### Definitions:

The term "absorbent article" herein refers to devices which absorb and contain body exudates and, more specifically, refers to devices which are placed against or in proximity to the body of the wearer to absorb and contain the various exudates discharged from the body, such as: incontinence briefs, incontinence undergarments, absorbent inserts, diaper holders and liners, feminine hygiene garments and the like. An absorbent article may have an absorbent core having a garment surface and a body facing surface, a liquid permeable topsheet positioned adjacent the body surface of the absorbent core, and a liquid impermeable backsheet positioned adjacent the garment facing surface of the absorbent core.

The term "disposable" is used herein to describe absorbent articles which generally are not intended to be laundered or otherwise restored or reused as absorbent articles (i.e., they are intended to be discarded after a single use and, preferably, to be recycled, composted or otherwise discarded in an environmentally compatible manner).

The term "diaper" herein refers to an absorbent article generally worn by infants and incontinent persons about the lower torso.

The term "pant", as used herein, refers to disposable garments having a waist opening and leg openings designed for infant or adult wearers. A pant may be placed in position on the wearer by inserting the wearer's legs into the leg openings and sliding the pant into position about the wearer's lower torso. A pant may be preformed by any suitable technique including, but not limited to, joining together portions of the article using refastenable and/or non-refastenable bonds (e.g., seam, weld, adhesive, cohesive bond, fastener, etc.). A pant may be preformed anywhere along the circumference of the article (e.g., side fastened, front waist fastened). While the term "pant" is used herein, pants are also commonly referred to as "closed diapers", "prefastened diapers", "pull-on diapers", "training pants" and "diaper-pants". Suitable pants are disclosed in U.S. Patent No. 5,246,433, issued to Hasse, et al. on September 21, 1993; U.S. Patent No. 5,569,234, issued to Buell et al. on October 29, 1996; U.S. Patent No. 6,120,487, issued to Ashton on September 19, 2000; U.S. Patent No. 6,120,489, issued to Johnson et al. on September 19, 2000; U.S. Patent No. 4,940,464, issued to Van Gompel et al. on July 10, 1990; U.S. Patent No. 5,092,861, issued to Nomura et al. on March 3, 1992; U.S. Patent Application Serial No. 10/171,249, entitled "Highly Flexible And Low Deformation Fastening Device", filed on June 13, 2002; U.S. Patent No. 5,897,545, issued to Kline et al. on April 27, 1999; U.S. Patent No. 5,957,908, issued to Kline et al on September 28, 1999.

The term "machine direction (MD)" or "longitudinal" herein refers to a direction running parallel to the maximum linear dimension of the article and/or fastening material and includes directions within ±45° of the longitudinal direction.

The term "cross direction (CD)", "lateral" or "transverse" herein refers to a direction which is orthogonal to the longitudinal direction.

The term "joined" encompasses configurations whereby an element is directly secured to another element by affixing the element directly to the other element, and configurations whereby an element is indirectly secured to another element by affixing the element to intermediate member(s) which in turn are affixed to the other element.

### Description:

### Fastening member.

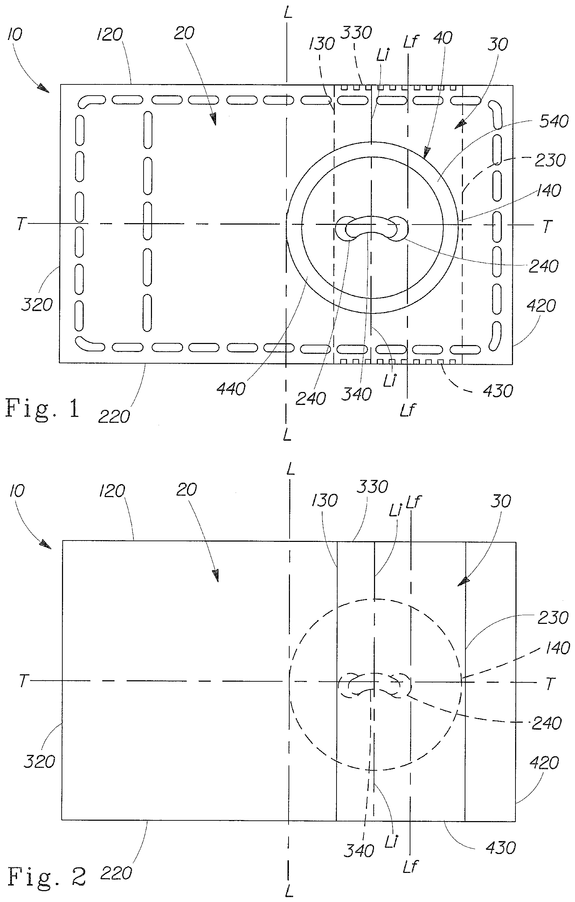

Figures 1 and 2 illustrate a fastening member 10 according to one embodiment of the invention, It should be noted that in Figure 1, the garment facing surface of the fastening member 10 (i.e. the surface that faces garment when the absorbent article is worn) faces the viewer. Conversely, it should be noted that in Figure 2, the body facing surface of the fastening member 10 (i.e. the surface that faces the body when the absorbent article is worn) faces the viewer. The fastening member 10 includes a tape 20 having a longitudinal axis L and transverse axis T. The tape 20 includes first and second transverse edges 120, 220 and first and second longitudinal edges 320, 420. The portion of the tape in the vicinity of the longitudinal edge 320 is the portion that is joined to the absorbent article. The fastening member 10 also includes an actual fastener 30 that is joined to one of the body or garment facings surface of the fastening member 10. In one embodiment, the actual fastener is disposed on the body facing surface of the fastening member 10. The fastener 30 has a longitudinal axis Lf and a transverse axis. The transverse axis of the fastener 30 illustrated in Figures 1 and 2, coincides with the transverse axis T of the tape 20. But embodiments where the transverse axis of the fastener does not coincide with the transverse axis T of the tape 20 are also contemplated. The fastener 30 has first and second longitudinal edges 130, 230 and first and second transverse edges 330, 430. The transverse edges 330, 430 of the fastener 30 represented in Figures 1 and 2 coincide with the transverse edges 120, 220 of the tape 20. It should be noted that embodiments where one or both transverse edges 330, 430 do not coincide with transverse edges 120, 220 of the tape are also within the scope of the invention. The longitudinal edge 230 of the fastener 30 is disposed inboard of the longitudinal edge 420 of the tape. Among other benefits, by disposing a longitudinal edge 230 of the fastener 30 inboard of the longitudinal edge 420 of the tape, the portion of the tape 20 that is located between the longitudinal edge 230 and the longitudinal edge 420, is free of fastener, which allows a care giver to peel the tape easily by grabbing this portion of tape. The fastener 30 may have any suitable transverse length. In the embodiment illustrated in Figures 1 and 2, the longitudinal edge 130 of the fastener 30 is disposed between the longitudinal axis L of the tape. The fastener 30 may be any known suitable fastener such as a layer adhesive, a layer cohesive or a mechanical fastener. It can be advantageous to have the fastener 30 in the form of a plurality of micro-mechanical fasteners such as hooks, prongs or mushroom. Non-limiting examples of such micro-mechanical fasteners are available from 3M, VELCRO or Aplix and typically include a base or film with a plurality of engaging elements projecting away from the base. In one embodiment, the base of the micro-mechanical fastener is joined to the body facing surface of the tape 20 such that the engaging elements projects away from the body facing surface of the tape 20. Each of the engaging elements is adapted to engage one or more of the fibers present on the surface of a receiving member (also referred as a landing zone), which can be made of a knitted or nonwoven fabric. The base of the micro-mechanical fastener can have a thickness of les than 2 mm, less than 1 mm or even less than 0.5 mm for increased flexibility. The engaging elements projecting from the base may have a height (measured from the base to the tip of the engaging element) of between 0.1 mm and 5 mm, between 0.1 mm and 2.5 mm or even between 0.1 mm and 1 mm. The number of engaging elements present on the fastener can be at least 10, at least 25, at least 50 or even at least 100 per cm² of micro-mechanical fastener for increased engagement with a complementary fibrous surface.

The fastening member 10 includes an indicium 40 that is visible on or through the garment facing surface of the tape 20. In one embodiment, the indicium 40 replicates the outer visible surface of macro-mechanical fastener that is commonly used in actual clothing articles. For the sake of clarity, the macro-mechanical fastener indicium (ia) 40 described herein is (are) not an actual fastener and is not capable by itself of being fastened to a button loop or buttonhole. The macro-mechanical fastener indicium 40 can be chosen from at least one of a sew-through button, a shaft or shank button (that may or may not be covered by a aesthetically pleasing fabric), and a push or snap button. The macro-mechanical fastener indicium 40 represented in Figure 1 is a sew-through button that has a substantially circular outer edge 140, two holes 240 and a thread 340 going from one of the holes to the other hole. The macro-mechanical fastener indicium 40 has a transverse axis, which coincides with the transverse axis T of the fastening member. It should be noted however that embodiments where the transverse axis of the macro-mechanical fastener indicium 40 does not coincide (i.e. is offset) with the transverse axis T of the fastening member 10 are also within the scope of the invention. The macro-mechanical fastener indicium 40 has a longitudinal axis Li. The macro-mechanical fastener indicium 40 can be disposed anywhere on the fastening member 10 as long as it is disposed in accordance with claim 1. When at least a portion of, or the whole fastener 30 is disposed on the body facing surface of the tape 20 between the longitudinal axis L of the fastening member and the longitudinal edge 420 of the tape 20, it can be advantageous to position the macro-mechanical fastener indicium 40 such that the longitudinal axis Li of the macro-mechanical fastener indicium 40 is present between the longitudinal axis L of the fastening member 10 and the longitudinal edge 420 of the tape 20. Without intending to be bound by any theory, it is believed that the macro-mechanical fastener indicium 40 visible from the garment facing surface of the tape 20, helps a care giver identify the position of a fastener on the fastening member for its proper placement and further engagement to the complementary fastening surface or receiving member. As is illustrated in Figures 1 and 2, the macro-mechanical fastener indicium 40 is positioned on the fastening member such that the longitudinal axis Li of the macro-mechanical fastener indicium 40 is disposed between the longitudinal axis L of the fastening member and the longitudinal axis Lf of the fastener 30. As a result of the positioning of the macro-mechanical fastener indicium 40 according to the present invention, at least a portion of the projected outer contour of macro-mechanical fastener indicium 40 intersects the inner longitudinal edge 130 of the fastener 30. Among other benefits, it is believed that a care giver is less likely to "miss" the corresponding fastening surface by using the macro-mechanical fastener indicium 40 previously discussed as a guide in particular when the corresponding fastening surface is narrower than the waist portion of the absorbent article as further discussed below. The macro-mechanical fastener indicium 40 may have any shape and size suitable to help and guide the care giver in positioning the fastener on the complementary fastening surface. The inner surface area (i.e. within the outer edge 140) can be at least 5 mm², at least 15 mm², at least 25 mm², or greater. At least a portion of the macro-mechanical fastener indicium 40 within the outer edge 140 include one or more colors and/or gradients of one or more colors, which distinguish the colored portion(s) from the tape 20. In one embodiment, the macro-mechanical fastener indicium 40 is asymmetric relative to its longitudinal axis Li and includes a portion 440 that is darker than a portion 540. Among other benefits, a portion 440 that is darker than a portion 540 and positioned on the macro-mechanical fastener indicium 40 such that each portion 440, 540 is disposed on respective sides of the longitudinal axis Li, replicate the effect a natural light source would have and enhances the three-dimensional appearance of the macro-mechanical fastener indicium 40. In one embodiment, the macro-mechanical fastener indicium 40 is asymmetric relative to its transverse axis and includes a portion 640 disposed on one of the sides of the macro-mechanical fastener indicium 40 relative to its transverse axis. The portion 640 can have a light color that provides a sharp contrast with the portions of the macro-mechanical fastener indicium 40 immediately adjacent the portion 640 and also provides a sharp contrast with portions of the macro-mechanical fastener indicium 40 disposed on the other side of its transverse axis. Among other benefits, the portion 640 replicates light reflection on an actual macro-mechanical fastener.

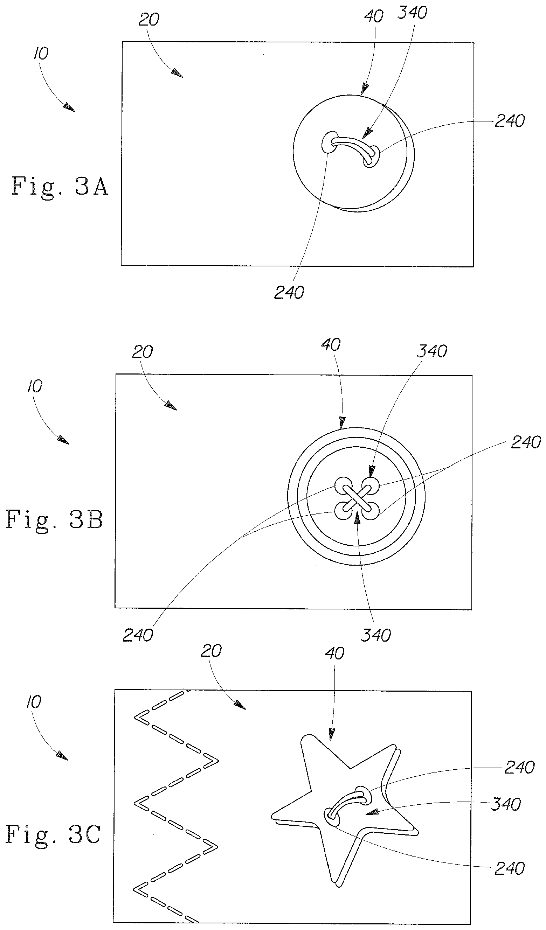

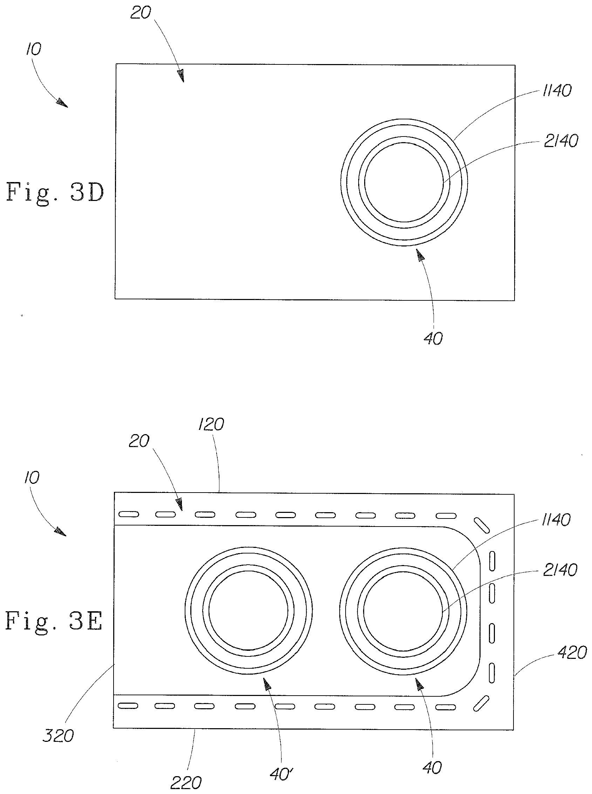

Figures 3A-3E illustrate alternative macro-mechanical fastener indicia. The macro-mechanical fastener indicium 40 represented in Fig. 3A replicates a sew-through button having a substantially flat outer surface and two holes 240, two threads 340 extending between the holes 340. It should be noted that in this embodiment, the holes 240 are not parallel to the transverse axis of the fastening member 10. The macro-mechanical fastener indicium 40 represented in Fig. 3B replicates a sew-through button having a three-dimensional outer surface, four holes 240 and two threads 340 extending between respective pairs of holes 340. The macro-mechanical fastener indicium 40 represented in Fig. 3C replicates a sew-through button having a three-dimensional outer surface, two holes 240 and two threads 340 extending between the two holes 340. The macro-mechanical fastener indicium 40 illustrated in Fig. 3C has the outer contour and shape of a star. It should be noted that although many actual sew-through button used on clothing have a disc shape, any other shape such as rectangular, triangular or other more complex shapes are also within the scope of the invention. The macro-mechanical fastener indicium 40 represented in Fig. 3D replicates a snap button (also called a push button) having a three-dimensional outer surface, an outer contour 1140 and an inner contour 2140. In can be advantageous to have the area of the tape located inside the inner contour 2140 of substantially a same color as the area of the tape located outside the outer contour 1140 to emphasize the presence of the macro-mechanical fastener indicium 40. Fig. 3E illustrate a fastening member including first and second macro-mechanical fastener indicia 40 and 40'. The macro-mechanical fastener indicium 40' is substantially identical in appearance to the macro-mechanical fastener indicium 40. But it should be understood that a fastening member having a plurality of macro-mechanical fastener indicia having different appearance is also within the scope of the invention. For example, a fastening member may include a first macro-mechanical fastener indicium replicating a macro fastener having a given appearance and a second macro-mechanical fastener indicium replicating a macro fastener having a different appearance such as via its shape, size, color and/or functionality (e.g. sew-through v. push button).

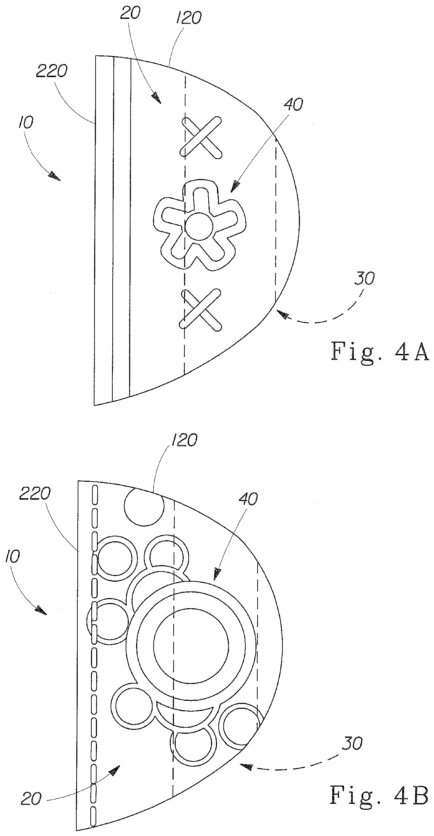

It will be appreciated that although the fastening members represented in Figures 1 through 4E have a substantially rectangular shape, the fastening member may have any suitable shape. For example, the fastening members represented in Figure 4A and 4B have an arc-like edge 120 and a longitudinal edge 220

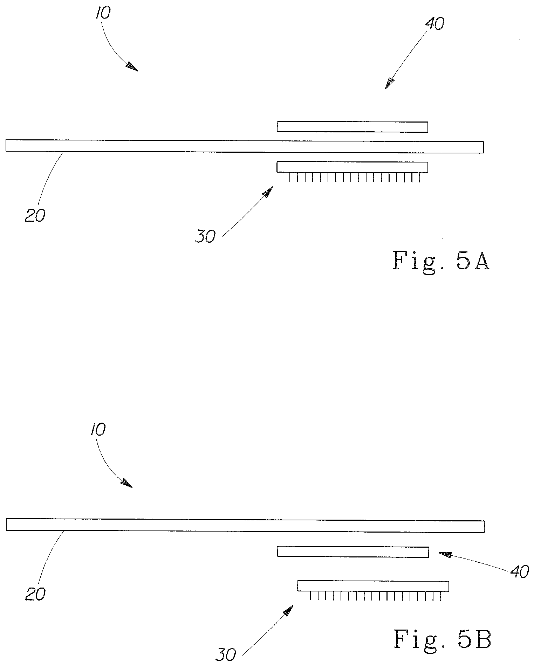

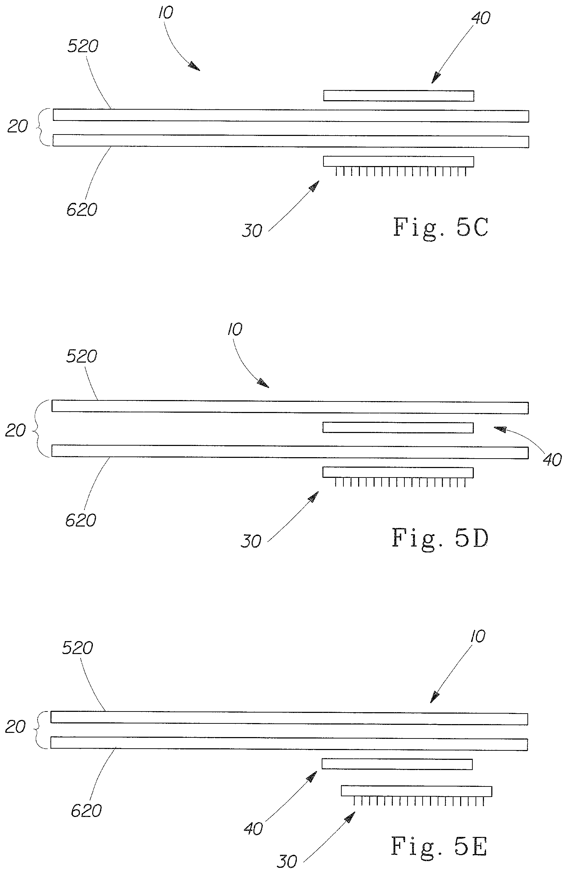

As previously discussed, it can be advantageous for a macro-mechanical fastener indicium 40 to be visible from or through the garment facing surface of a fastening member. A macro-mechanical fastener indicium 40 can be provided on a fastening member by printing the substrate forming for example the tape with one or more inks. It should be noted that when rubbing-off of the ink is a concern, inks that have good crockfastness resistance may be employed. In the alternative, inks with lower crockfastness may also be employed but it can be benefitial to cover the printed area with a lacquer. Cross-sectional views of fastening members (taken along transverse axis T) having a macro-mechanical fastener indicium 40 are schematically represented in Figures 5A-5E. In one embodiment, a macro-mechanical fastener indicium 40 is disposed on the garment facing surface of the tape 20 as illustrated in Figure 5A. In another embodiment illustrated in Figure 5B, a macro-mechanical fastener indicium 40 is disposed on the body facing surface of the tape 20 such that the fastener 30 covers at least partially or even in its entirety the macro-mechanical fastener indicium 40. A fastener 30 covering at least a portion of the macro-mechanical fastener indicium 40 reduces the chances the indicium may rub-off due to chemical or mechanical action. Such an embodiment can be advantageous when the indicium is printed via an ink that tends to rub-off easily. If the size of macro-mechanical fastener indicium 40 is equal or less than the width of the fastener 30, the macro-mechanical fastener indicium 40 can also be disposed on a back surface of the fastener (i.e. the surface opposite to the surface from which the engaging elements projects). Although the tape 20 illustrated in Figures 5A and 5B includes a single layer of material, it is understood that embodiments where the tape 20 includes more than a single layer of material are also within the scope of the invention. For example, the tape 20 can include two or more layers of materials that are laminated to each other to form a tape. In the embodiments illustrated in Figures 5C-5D, the tape 20 includes a first layer of material 520, which is joined to a second layer of material 620. In one embodiment, the first layer of material 520 is one of a fibrous nonwoven material and a film, and the second layer 620 is one of a fibrous nonwoven material and a film. In the embodiment illustrated in Figure 5C, the macro-mechanical fastener indicium 40 is present on the outer surface of the first layer of material 520, which forms the garment facing surface of the fastening member. In the embodiment illustrated in Figure 5D, the macro-mechanical fastener indicium 40 is present on the inner surface of the first layer of material 520, and is nevertheless visible from garment facing surface of the fastening member. The macro-mechanical fastener indicium 40 can also be present on the inner surface of the second layer of material 620 and is then covered by the first layer of material 520. In the embodiment illustrated in Figure 5E, the macro-mechanical fastener indicium 40 is present on the outer surface of the second layer of material 620, and is nevertheless visible from garment facing surface of the fastening member. As previously discussed, positioning the macro-mechanical fastener indicium 40 on a surface other than the outermost surface of the tape can prevent the macro-mechanical fastener indicium 40 from rubbing-off due to mechanical or chemical action. The tape 20 can include additional layers of material such as at least one additional layer of a fibrous nonwoven material such that the tape forms a laminate of garment facing fibrous nonwoven, a body facing nonwoven and a film disposed between the garment facing and the body facing fibrous nonwoven materials.

### Absorbent article.

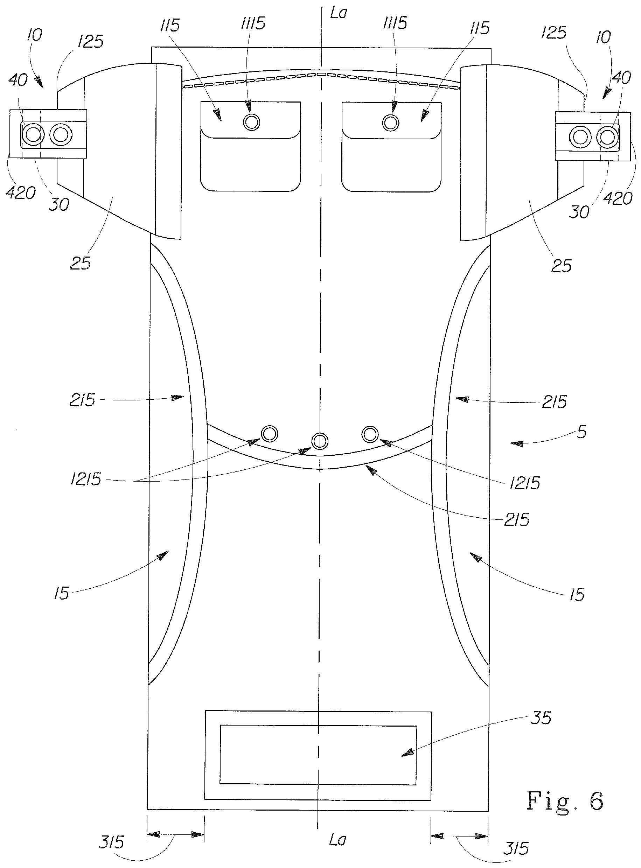

As previously discussed, a fastening member with an indicium, which is visible can help a care giver identify the position of a fastener for its proper engagement to the complementary fastening surface or receiving member. This can be accomplished by incorporating any of the previously discussed fastening members to an absorbent article while improving the aesthetic and garment like appearance of the disposable absorbent article. The garment facing side of an absorbent article 5 in a flat out state is represented in Figure 6. The absorbent article 5 includes a liquid pervious topsheet (not shown), a liquid impervious backsheet 15 and an absorbent core (not shown). The absorbent article may include a pair of side panels 25, which are joined to a waist region of the absorbent article as is well know in the art. The side panels 25 can be substantially inelastic but it can be advantageous to provide the absorbent article with elastically extensible side panel. The absorbent article 5 includes a pair of fastening members 10 that are joined to a distal portion of the respective side panels 25. The absorbent article can also include a receiving member 35 joined to the opposite waist region of the absorbent article. The receiving member 35 provides a complementary surface, which is suitable to interact with the fastening members 10 in order to attach the absorbent article about the lower torso of a wearer. In one embodiment, the receiving member 35 is joined to the backsheet 15 of the article such that the receiving member 35 is substantially centered about the longitudinal axis La of the article 5. In one embodiment, the transverse length of the receiving member is less than the transverse length of the absorbent article. As a result, the article can include two portions 315 on each transverse side of the receiving member 35 that are not adapted or less adapted to interact with the fastener of the fastening members 10. One skill in the art will understand that the smaller the receiving member is then the larger these portions 315 become and the greater the risk a care giver may not apply the fasteners to the receiving member 35. The usefulness of the embodiments of the invention becomes even more apparent when the distance between the transverse edge of the receiving member and the transverse edge of the absorbent article is greater than the distance between the distal transverse edge 125 of the side panels 25 and the transverse edge 420 of the fastening member 10. Each indicium, and in particular macro-mechanical fastener indicium 40, can be used as a guide that helps the care giver position the fastener (less visible to the care giver) on the receiving member. In order to enhance the garment-like appearance of the absorbent article and also communicate the usefulness of the indicia on the fastening member, it can be advantageous to provide the backsheet 15 with additional indicia. In one embodiment, the backsheet includes one or more indicium 115 that replicate a pocket such as the pocket found in an actual piece of clothing. A pocket indicium can include a macro-mechanical fastener indicium 1115 that is similar to the macro-mechanical fastener indicium 40 present on the fastening member 10. The backsheet 15 can also include other garment-like attributes such as seams 215 that replicate actual seams that are typically present on a piece of clothing. It can also be advantageous to include one or more macro-mechanical fastener indicium 1215 in the proximity of at least one of the seam indicium 215 to replicate separate portions of clothing that are fastened together and are present in the crotch portion of a wearer.

### Process/method of making.

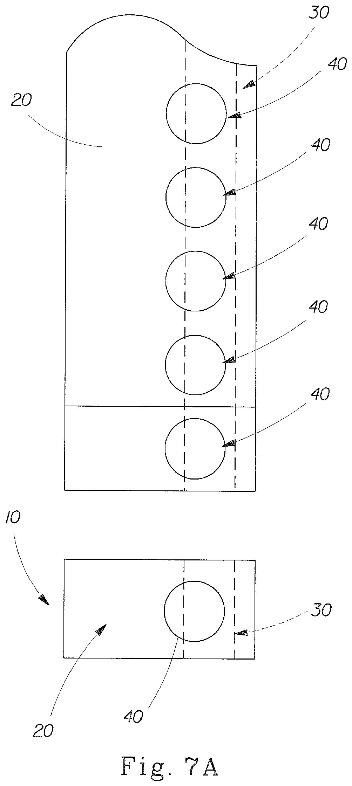

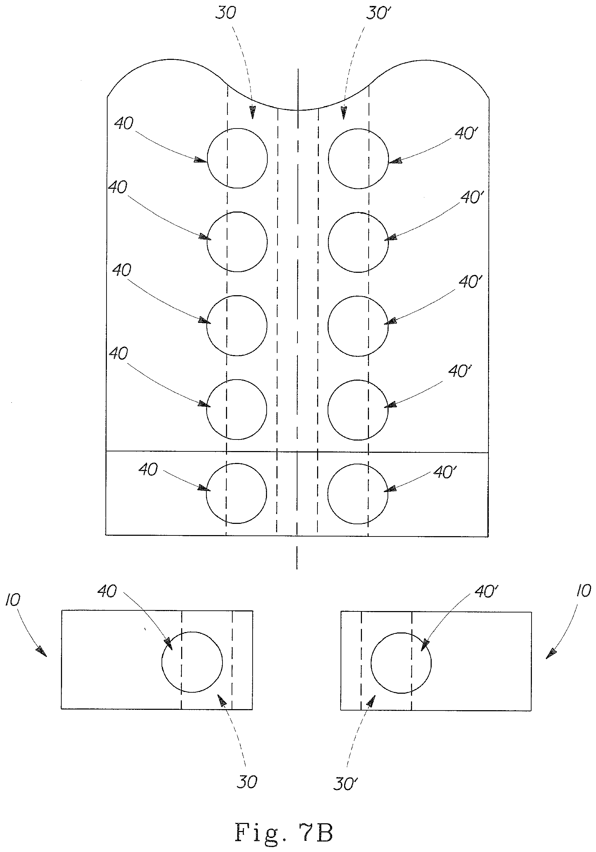

The fastening members previously described can be obtained by cutting a plurality of individual fastening members 10 from a continuous (i.e. long length) strip material. Such continuous strip of material can be in the form of a roll that is unwound and then cut. The continuous strip of material can include one or more layer forming the tape portion of the fastening member, at least one continuous strip of fastener material disposed on one side of the continuous material forming the tape and a plurality of individual indicia (such as the macro-mechanical fastener indicia previous described) that are distant and separate from each other. Although the particular location of the transverse cut has no impact on the continuous strips of materials forming the tape portion and the fastener of the fastening member, it is beneficial to cut the continuous strip such that at least one macro-mechanical fastener indicium 40 is present in its entirety on the resulting individual fastening members 10 as schematically illustrated in Figure 7A. In one embodiment schematically illustrated in Figure 7B, it can be advantageous to cut a plurality of individual fastening members 10 from a continuous strip of material including a continuous strip of material suitable to form the tape portion 20 of the fastening member and two or more continuous strips of fastener materials 30, 30'. The two continuous strips of fastener material are joined to the continuous strip of material suitable to form the tape such that strips of fastener material are separated from each other by a distance sufficient to provide a grabbing portion on respective left and right fastening members. The continuous strip of fastening member material includes a first plurality of individual macro-mechanical fastener indicium 40 and at least a second plurality of individual macro-mechanical fastener indicium 40'. In the embodiment illustrated in Figure 7B, the second plurality of individual macro-mechanical fastener indicium 40' is the mirror image of the first plurality of individual macro-mechanical fastener indicium 40' relative to the longitudinal axis of the continuous strip of fastening member material.

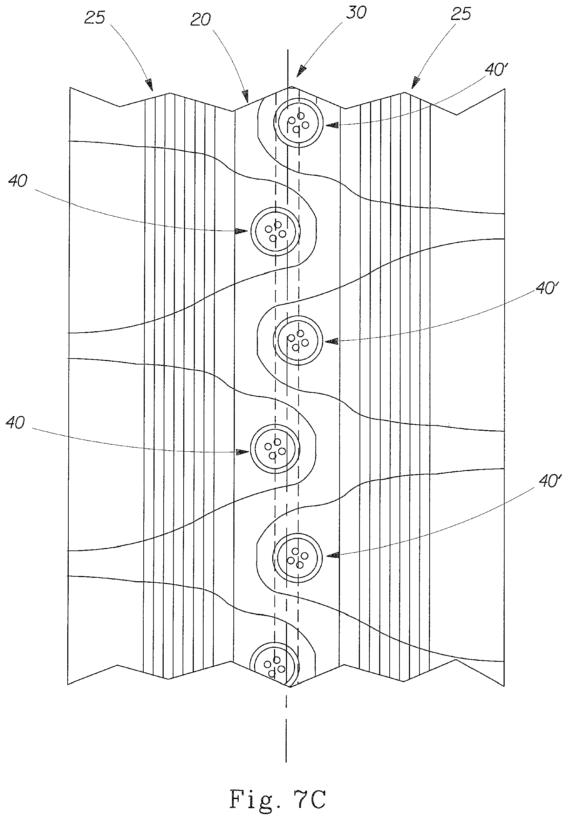

As previously discussed, the shape of the fastening member 10 can be, but is not necessarily, rectangular. A process of cutting individual and separate fastening members having an arc-like edge is schematically illustrated in Figure 7C. It should be noted that in this embodiment, the fastening member from the cut is integral with the side panel material 25. But individual fastening members may also be obtained by this process. A continuous strip of fastening member material includes a continuous strip of tape-like portion material 20, a continuous strip of fastener material 30, a first plurality of individual macro-mechanical fastener indicium 40 and a second plurality of individual macro-mechanical fastener indicium 40'. Due to the more complex shape of the fastening members, it can be advantageous to dispose the plurality macro-mechanical fastener indicia 40, 40' such that the first plurality of individual macro-mechanical fastener indicium 40 and the second plurality of individual macro-mechanical fastener indicium 40' are offset relative to the longitudinal axis of the continuous materials (i.e. offset in the cross direction). When the individual macro-mechanical fastener indicia are offset, each fastening member can be "nested," which minimizes the amount of waste.

Although the individual macro-mechanical fastener indicia may be printed before or after the continuous strip of fastener material 30 is joined to the other materials, it can be beneficial to print the individual macro-mechanical fastener indicia 40 before the continuous strip of fastener material 30 is joined to the printed continuous strip of material 20. Without intending to be bound by any theory, it is believed that printing the indicia before joining the fastener material 30 minimizes potential damage or contamination of the fastener material, which may occur if the fastener material is joined to the material 20 before it is printed.

## Claims

1. An absorbent article (5) having a liquid pervious topsheet, a backsheet (15) and an absorbent core disposed between the topsheet and the backsheet (15), a longitudinal axis (La) and a transverse axis, the absorbent article (5) being **characterized in that** it comprises:
at least one fastening member (10), the fastening member (10) having longitudinal (L) and transverse (T) axes, said fastening member (10) comprising:
a tape (20) having a garment facing surface and a body facing surface;
an actual fastener (30) disposed on said tape (20), wherein said actual fastener (30) is adapted to engage a complementary surface; and
a macro-mechanical fastener indicium (40) that is visible on or through the garment facing surface of the tape (20), wherein said fastener (30) comprises inner and outer longitudinal edges (130, 230) and said macro-mechanical fastener indicium (40) comprises a contour,
wherein said macro-mechanical fastener indicium (40) is disposed on said fastening member (10) such that at least a portion of the projected outer contour of the macro-mechanical fastener indicium (40) intersects said inner longitudinal edge (130) of said fastener (30),
and further wherein that said tape (20) comprises a first longitudinal edge (320), a second longitudinal edge (420) and a longitudinal axis (L), wherein said fastener (30) comprises an inner longitudinal edge (130), an outer longitudinal edge (230) and wherein said fastener (30) is joined to the body facing surface of said tape (20) such that the longitudinal axis (Lf) of said fastener (30) is disposed between the longitudinal axis (L) and the second edge (420) of said tape (20), and wherein said macromechanical fastener indicium (40) comprising a contour and a longitudinal axis (Li) wherein said macromechanical fastener indicium (40) is disposed on said fastening member (30) such that the longitudinal axis (Li) of said macro-mechanical fastener indicium (40) is located between the first longitudinal edge (320) of said tape (20) and said longitudinal axis (Lf) of said fastener (30) and said macro-mechanical fastener indicium (40) is located between said longitudinal axis (L) of said tape (20) and said longitudinal axis (Lf) of said fastener (30),
wherein the outer longitudinal edge (230) of the fastener (30) is disposed inboard of the second longitudinal edge (420) of the tape.

2. The absorbent article (5) according to claim 1 **characterized in that** said fastener (30) comprises micro-mechanical fastener comprising a base and a plurality of engaging elements wherein said base is joined to the body facing surface of said tape (20) such that said plurality of engaging elements project away from said body facing surface of said tape (20).

3. The absorbent article (5) according to claim 1 **characterized in that** said macro-mechanical fastener indicium (40) is chosen from at least one of a sew-through button, a shaft button, and a push button.

4. The absorbent article (5) according to claim 1 **characterized in that** said macro-mechanical fastener indicium (40) is printed on said tape (20).

5. The absorbent article (5) according to claim 1 **characterized in that** said tape (20) comprises a first layer of material (520) joined to a second layer of material (620) and wherein said macro-mechanical fastener indicium (40) is printed on one of said first and second layers of material (520, 620).

6. The absorbent article (5) according to claim 8 **characterized in that** said macro-mechanical fastener indicium (40) is disposed between said first and second layers of material (520, 620).

## Patentansprüche

1. Absorptionsartikel (5) mit einer flüssigkeitsdurchlässigen Oberschicht, einer Unterschicht (15) und einem zwischen der Oberschicht und der Unterschicht (15) angeordneten Absorptionskern, einer Längsachse (La) und einer Querachse, wobei der Absorptionsartikel (5) **dadurch gekennzeichnet ist, dass** er Folgendes umfasst:
mindestens ein Befestigungselement (10), wobei das Befestigungselement (10) eine Längsachse (L) und eine Querachse (T) aufweist, wobei das Befestigungselement (10) Folgendes umfasst:
ein Band (20) mit einer bekleidungsseitigen Oberfläche und einer körperseitigen Oberfläche;
einen tatsächlichen Verschluss (30), der auf dem Band (20) angeordnet ist, wobei der tatsächliche Verschluss (30) so konzipiert ist, dass er mit einer komplementären Oberfläche ineinandergreift; und
eine makromechanische Verschlussmarkierung (40), die auf der bekleidungsseitigen Oberfläche des Bands (20) oder durch diese hindurch sichtbar ist, wobei der Verschluss (30) innere und äußere Längsränder (130, 230) umfasst und wobei die makromechanische Verschlussmarkierung (40) eine Kontur umfasst, wobei die makromechanische Verschlussmarkierung (40) derart auf dem Befestigungselement (10) angeordnet ist, dass wenigstens ein Abschnitt der projizierten Außenkontur der makromechanischen Verschlussmarkierung (40) den inneren Längsrand (130) des Verschlusses (30) schneidet,
und wobei ferner das Band (20) einen ersten Längsrand (320), einen zweiten Längsrand (420) und eine Längsachse (L) umfasst, wobei der Verschluss (30) einen inneren Längsrand (130) und einen äußeren Längsrand (230) umfasst und wobei der Verschluss (30) derart an der körperseitigen Oberfläche des Bands (20) angebracht ist, dass die Längsachse (Lf) des Verschlusses (30) zwischen der Längsachse (L) und dem zweiten Rand (420) des Bands (20) angeordnet ist, und wobei die makromechanische Verschlussmarkierung (40) eine Kontur und eine Längsachse (Li) umfasst, wobei die makromechanische Verschlussmarkierung (40) derart auf dem Befestigungselement (30) angeordnet ist, dass sich die Längsachse (Li) der makromechanischen Verschlussmarkierung (40) zwischen dem ersten Längsrand (320) des Bands (20) und der Längsachse (Lf) des Verschlusses (30) befindet und sich die makromechanische Verschlussmarkierung (40) zwischen der Längsachse (L) des Bands (20) und der Längsachse (Lf) des Verschlusses (30) befindet,
wobei der äußere Längsrand (230) des Verschlusses (30) innerhalb des zweiten Längsrands (420) des Bands angeordnet ist.

2. Absorptionsartikel (5) nach Anspruch 1, **dadurch gekennzeichnet, dass** der Verschluss (30) mikromechanischen Verschluss umfasst, der eine Basis und eine Vielzahl von eingreifenden Elementen umfasst, wobei die Basis derart an der körperseitigen Oberfläche des Bands (20) befestigt ist, dass die Vielzahl von eingreifenden Elementen von der körperseitigen Oberfläche des Bands (20) wegweist.

3. Absorptionsartikel (5) nach Anspruch 1, **dadurch gekennzeichnet, dass** die makromechanische Verschlussmarkierung (40) aus wenigstens einem von einem durchsichtigen Knopf, einem Schaftknopf und einem Druckknopf ausgewählt ist.

4. Absorptionsartikel (5) nach Anspruch 1, **dadurch gekennzeichnet, dass** die makromechanische Verschlussmarkierung (40) auf das Band (20) gedruckt ist.

5. Absorptionsartikel (5) nach Anspruch 1. **dadurch gekennzeichnet, dass** das Band (20) eine erste Materialschicht (520) umfasst, die an einer zweiten Materialschicht (620) befestigt ist und wobei die makromechanische Verschlussmarkierung (40) auf eine der ersten oder zweiten Materialschichten (520, 620) gedruckt ist.

6. Absorptionsartikel (5) nach Anspruch 8, **dadurch gekennzeichnet, dass** die makromechanische Verschlussmarkierung (40) zwischen der ersten und zweiten Materialschicht (520, 620) angeordnet ist.

## Revendications

1. Article absorbant (5) ayant une feuille de dessus perméable aux liquides, une feuille de fond (15) et une âme absorbante disposée entre la feuille de dessus et la feuille de fond (15), un axe longitudinal (La) et un axe transversal, l'article absorbant (5) étant **caractérisé en ce qu'**il comprend :
au moins un élément de fixation (10), l'élément de fixation (10) ayant des axes longitudinal (L) et transversal (T), ledit élément de fixation (10) comprenant :
un ruban (20) ayant une surface tournée vers le vêtement et une surface tournée vers le corps ;
un fermoir réel (30) disposé sur ledit ruban (20), dans lequel ledit fermoir réel (30) est conçu pour venir en prise avec une surface complémentaire ; et
une indication de fermoir macromécanique (40) qui est visible sur ou à travers la surface tournée vers le vêtement du ruban (20), dans lequel ledit fermoir (30) comprend des bords longitudinaux interne et externe (130, 230) et ladite indication de fermoir macromécanique (40) comprend un contour, dans lequel ladite indication de fermoir macromécanique (40) est disposée sur ledit élément de fixation (10) de telle sorte qu'au moins une partie du contour externe projeté de l'indication de fermoir macromécanique (40) croise ledit bord longitudinal interne (130) dudit fermoir (30),
et dans lequel en outre ledit ruban (20) comprend un premier bord longitudinal (320), un deuxième bord longitudinal (420) et un axe longitudinal (L), dans lequel ledit fermoir (30) comprend un bord longitudinal interne (130), un bord longitudinal externe (230) et dans lequel ledit fermoir (30) est joint à la surface tournée vers le corps dudit ruban (20) de telle sorte que l'axe longitudinal (Lf) dudit fermoir (30) est disposé entre l'axe longitudinal (L) et le deuxième bord (420) dudit ruban (20), et dans lequel ladite indication de fermoir macromécanique (40) comprend un contour et un axe longitudinal (Li) dans lequel ladite indication de fermoir macromécanique (40) est disposée sur ledit élément de fixation (30) de telle sorte que l'axe longitudinal (Li) de ladite indication de fermoir macromécanique (40) est située entre le premier bord longitudinal (320) dudit ruban (20) et ledit axe longitudinal (Lf) dudit fermoir (30) et ladite indication de fermoir macromécanique (40) est située entre ledit axe longitudinal (L) dudit ruban (20) et ledit axe longitudinal (Lf) dudit fermoir (30),
dans lequel le bord longitudinal externe (230) du fermoir (30) est disposé à l'intérieur du deuxième bord longitudinal (420) du ruban.

2. Article absorbant (5) selon la revendication 1, **caractérisé en ce que** ledit fermoir (30) comprend un fermoir micro-mécanique comprenant une base et une pluralité d'éléments d'accrochage, dans lequel ladite base est jointe à la surface tournée vers le corps dudit ruban (20) de telle sorte que ladite pluralité d'éléments d'accrochage fait saillie à l'écart de ladite surface tournée vers le corps dudit ruban (20).

3. Article absorbant (5) selon la revendication 1, **caractérisé en ce que** ladite indication de fermoir macromécanique (40) est choisie parmi au moins l'un parmi un bouton cousu à travers, un bouton à tige et un bouton à pousser.

4. Article absorbant (5) selon la revendication 1 **caractérisé en ce que** ladite indication de fermoir macromécanique (40) est imprimée sur ledit ruban (20).

5. Article absorbant (5) selon la revendication 1, **caractérisé en ce que** ledit ruban (20) comprend une première couche de matériau (520) jointe à une deuxième couche de matériau (620) et dans lequel ladite indication de fermoir macromécanique (40) est imprimée sur l'une desdites première et deuxième couches de matériau (520, 620).

6. Article absorbant (5) selon la revendication 8, **caractérisé en ce que** ladite indication de fermoir macromécanique (40) est disposée entre lesdites première et deuxième couches de matériau (520, 620).
